Europäisches Patentamt

European Patent Office

Office européen des brevets

(19)

(11) Publication number: **0 258 870**
**A1**

(12) **EUROPEAN PATENT APPLICATION**

(21) Application number: 87112738.7

(51) Int. Cl.⁴: **B01J 29/28** , C07C 5/27

(22) Date of filing: 01.09.87

(30) Priority: 01.09.86 IL 79906

(43) Date of publication of application:
09.03.88 Bulletin 88/10

(84) Designated Contracting States:
AT BE CH DE ES FR GB GR IT LI LU NL SE

(71) Applicant: YEDA RESEARCH AND
DEVELOPMENT COMPANY, LIMITED
P.O. Box 95
Rehovot 76100(IL)

(72) Inventor: Fraenkel, Dan
9/1 Miller Street
Rehovot(IL)
Inventor: Levy, Moshe
42 Weizmann Street
Rehovot(IL)

(74) Representative: Vossius & Partner
Siebertstrasse 4 P.O. Box 86 07 67
D-8000 München 86(DE)

(54) Novel crystalline aluminosilicate zeolite catalysts and their use in the vapor phase isomerization of xylenes.

(57) The invention relates to novel crystalline aluminosilicate catalysts and to a process for the production of such catalysts. The invention further relates to such process where highly specific starting materials are reacted under closely controlled conditions of reaction to produce the desired products. Furthermore, the invention relates to a highly selective process for the isomerization of xylene by means of such catalyst. Starting materials comprising mainly m-xylene are readily converted to isomeric mixtures of xylene close to the equilibrium composition.

EP 0 258 870 A1

## Novel crystalline alumino-silicate zeolite catalysts and their use in the vapor phase isomerization of xylenes

### Field of the Invention:

The present invention relates to the conduction of selective vapor phase isomerization of xylene over crystalline alumino-silicate zeolite catalysts. Novel catalysts having improved properties are obtained from NaAlO$_2$-Pr$_4$-NOH-SiO$_2$-H$_2$O reactant combinations of a narrow range, where Pr designates propyl.

### Background of the Invention:

The catalytic rearrangement of alkyl groups present in alkyl aromatic hydrocarbons to provide one or more products suitable for use in the petroleum and chemical industries has heretofore been effected by a wide variety of catalysts. Acidic halides such as aluminum chloride, aluminum bromide, boron trifluoride - hydrogen fluoride mixtures, etc. have been used in the rearrangement of alkyl benzenes to provide valuable intermediates which find utility in the synthesis of rubber, plastics, fibers and dyes. Other catalysts which have been used include solid siliceous cracking-type catalysts such as silica-alumina and clays and platinum deposited on silica-alumina. Although various catalysts possess one or more desired characteristics, a majority of catalysts heretofore employed suffer form several disadvantages. Acidic halides such as aluminum chloride, for example, are partially soluble in the feed material and easily lost from the catalyst zone. Catalysts of this type are also uneconomical because of their extreme corrosiveness and requirement for recovery from the effluent products. Other catalysts of the heterogeneous type such as silica-alumina, platinum on alumina, etc., do not possess sufficient acidity to provide effective conversion and necessitate the use of relatively high temperatures above the order of 425°C to 510°C. High temperatures frequently lead to coke formation which lowers the yield of the desired product and necessitates frequent regeneration of the catalyst to remove coke. This results in reducing on-stream time and leads to high catalyst consumption due to loss of catalyst activity. Heterogeneous catalysts such as the crystalline aluminosilicates, both natural and synthetic, possess sufficient acidity but suffer from the disadvantage of poor selectivity and aging as evidenced by "coke" formation and the excessive amounts of disproportionated products formed in isomerization reactions.

A process in the art for isomerization of xylene of Octafining, extensively discussed in the literature as exemplified by: Ciapetta, F.G., Buck, W.H., U.S. Patent No. 2,589,189; Octafining Process, Process Issue, Petroleum Refinery, 1st. Vol. 38 (1959), No 11, Nov., p. 278. The catalyst used in this process is platinum on silica-alumina.

An improved catalyst for use in Octafining plants is taught by U.S. Patent No. 3,856,872 to be of the ZSM-5 type of zeolite, where the process operates at high space velocities.

Even in such an improved process, especially when the catalyst has increased acid activity resulting from decreasing intracrystalline diffusional resistance, there is a loss of xylene, presumably due to disproportionation of xylenes and transalkylation of xylenes with ethylbenzene. A metal of Group VIII of the Periodic Table of Elements is incorporated in the ZSM-5 containing catalyst, primarily as a hydrogenation component that in the presence of hydrogen will inhibit coke formation and reduce aging. By increasing the Group VIII metal content of such a catalyst to an established minimum balance between the hydrogenation activity of the Group VIII metal and the acid activity of the HZSM-5 permits greater hydrodealkylation, particularly deethylation of the C$_9^+$ fraction formed as demonstrated in U.S. Patent No. 4,098,836. Since a good portion of the C$_9^+$ fraction is dimethylethylbenzene, formed by the transalkylation of xylene with ethylbenzene, deethylation of this material would result in a recovery of product in the xylene fraction previously lost in the C$_9^+$ fraction. Furthermore, the improved deethylation activity of the catalysts with increased Group VIII metal content will increase the ethybenzene disappearance at the same severity with the concomitant advantage of higher value benzene byproduct rather than the heavy polyethylbenzenes.

Sumary of the invention :

The invention relates to a process of xylene isomerization performed under mild conditions (less than ~ 300°C) at low space velocities, in which nearly full xylene-isomerization equilibrium is attained practically without xylene degradation and ethyl-benzene conversion, and with minumum deethylation and gas formation. High conversion rates are obtained, and coke formation is prevented for a period of at least several months, as reflected by a nearly constant catalyst activity during months of continuous operation on a small scale. A xylene isomerization process as described herein requires frequent removal of ethylbenzene from recycle feed. The disadvantage of much lower benzene yield in the absence of extensive deethylation is more than compensated by suppressing C9+ and gas formation. The key factor in this process is a novel ZSM-5 type catalyst. U.S. Patent No. 3,702,8986 discloses the synthesis of ZSM-5 materials from a reactant mixture containing $Na_2O$, $Al_2O_3$, $Pr_4NOH$, $SiO_2$ and $H_2O$ (Pr = normal-propyl) but there is not provided any information on their performance as catalysts.

Moreover, no hint is given as to which particular catalytic process can be affected by the wide range of different catalysts obtainable according to this patent. Furthermore, the patent does not teach a reactant composition and reaction conditions which are appropriate for preparing an effective xylene isomerization catalyst. We have identified a narrow composition range within which the synthesis of ZSM-5 type materials can be carried out to yield products especially suited for the selective xylene isomerization under mild conditions.

Thus, this invention relates to the formation of an improved catalyst and to its use for vapor phase isomerization of monocyclic alkyl aromatic hydrocarbon feed. The isomerization reaction is carried out in the presence of a catalyst composition comprising a crystalline aluminosilicate zeolite characterized by a silica/alumina mole ratio greater than 12.

The crystalline aluminosilicate zeolites used as catalysts of the process of this invention are referred to generally as ZSM-5 type or as behaving like ZSM-5 and are represented by the general formula, expressed in terms of mole ratios of oxides in the anhydrous state, as follows:
ZSM-5
$(0.9 \pm 0.2)M_{2/n}O:Al_2O_3:xSiO_2$
wherein M is a cation such as an alkali metal cation, or proton, n is the valence of M and x is at least 5.

The catalyst contains $H^+$ ions produced either by treatment with an acid, normally a strong mineral acid like HCl, or indirectly by thermally decomposing the $NH_4^+$ form obtained by treating the "as synthesized" material with an ammonium salt solution, e.g., 1N NH4Cl in water.

The catalyst of the present invention is synthesized from reactant combinations in the range of
1.2 Na2O: 6 Pr4NOH: Al2O3: 40 SiO2: 280 H2O
The $SiO_2/Pr_4NOH$ molar ratio (~7) is about 4-fold larger than that used in typical ZSM-5 synthesis, such as that described in U.S. Patent No. 3,702,886. This ratio can be increased to ~ 13. The $H_2O/SiO_2$ ratio is ~7 and thus 2-fold smaller than that typically used, and it can be further reduced to 3.5.

Description of Preferred Embodiments:

The catalyst composition useful in this invention comprises a crystalline aluminosilicate zeolite characterized by a silica:alumina mole ratio of at least 12. Zeolite ZSM-5 is taught by U.S. Patent No. 3,702,886, issued Nov. 14, 1972, the disclosure of which is incorporated herein by reference. In terms of molar ratios of oxides in anhydrous state, it has the formula;
$(0.9 \pm 0.2) M_{2/n}O:Al_2O_3:xSiO_2$
wherein M is selected from a group consisting of a mixture of alkali metal cations, especially sodium, and tetraalkylammonium cations the alkyl groups of which preferably contain 2 to 5 carbon atoms, especially 3 carbon atoms, and x is at least 5 but preferably from greater than 30 to about 350 or higher.

Although the zeolites herein described have an unusually low alumina content, i.e. high silica to alumina ratios, they are very active even when the silica to alumina ratio exceeds 30. The activity is surprising since catalytic activity is generally attributed to framework aluminum atoms and to cations associated with these aluminum atoms. These catalysts retain their crystallinity for long periods in spite of the presence of steam at high temperature which induces irreversible collapse of the framework of other zeolites, e.g. of the X and A type. Furthermore, carbonaceous deposits, when formed, may be removed by burning at higher than usual temperatures to restore activity. In many environments the zeolites of this class exhibit very low coke forming capability, conducive to very long times on stream between burning regenerations.

An important characteristic of the crystal structure of the zeolites for use herein is that they provide constrained access to, and egress from, the intracrystalline free space by virtue of having a pore dimension greater than about 5 Angstroms and pore windows of about a size such as would be provided by 10-membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. Briefly, the preferred type catalysts useful in this invention possess, in combination: a silica to alumina ratio of at least about 12; and a structure providing constrained access to the crystalline free space.

The silica to alumina ratio referred to may be determined by conventional analysis. This ratio is meant to represent as closely as possible, the ratio in the rigid anionic framework of the zeolite crystal and to exclude aluminum in the binder or in cationic or other form within the channels. Although catalysts with a silica to alumina ratio of at least 12 are useful, it is preferred to use catalysts having higher ratios, of preferably about 30. Such catalysts, after activation, acquire an intracrystalline sorption capacity for normal hexane which is greater than that for water, i.e. they exhibit "hydrophobic" properties. It is believed that this hydrophobic character is advantageous in the present invention.

The present invention provides a highly effective vapor phase isomerization process with a ZSM-5 type catalyst, the crystalline aluminosilicate zeolite portion of which, as suggested above, has a smaller pore size than those crystalline aluminosilicates previously used for such purpose.

The specific zeolite described, when prepared in the presence of organic cations, are catalytically inactive, possibly because the intracrystalline free space in occupied by organic cations from the forming solution. They can be activated by heating, for example, in an inert atmosphere at 540°C for one hour, followed by base exchange with ammonium salts and by calcination at 540°C in air.

Typically, an effective xylene isomerization zeolite catalyst according to this embodiment is prepared by mixing a solution of sodium aluminate with a mixture of silica and $Pr_4NOH$ in water containing the appropriate solution and undissolved excess of the silica. The obtained mixture is allowed to age for between ~0.5 h and several hours, then put in the oven at a temperature in the range of about 90°C to about 180°C for a period of time between a few hours and a few weeks. A convenient way of treating the crystalline material obtained is evaporation of excess liquid at ~ 95°C then calcination at 540°C for~3 h before finally ion exchanging with ammonium chloride, washing free of chloride and drying at ~ 110°C for a few hours. The final calcination, to give the $H^+$ form by decomposing $NH_4^+$ , can be done in situ prior to reacting the catalyst with the xylene feed.

For the isomerization process of this invention the novel zeolite catalyst is employed in combination with a support or binder material such as, for example, a porous inorganic oxide carrier or a clay binder. Non-limiting examples of such binder materials include alumina, zirconia, silica, magnesia, thoria, titania, boria and combinations thereof, generally in the form of dried inorganic oxide gels and gelatinous precipitates. Suitable clay materials include, by way of example, bentonite and kieselguhr. The relative proportion of suitable crystalline aluminosilicate zeolite of the total composition of catalyst and binder or support may vary widely with the zeolite content ranging from between about 10 to about 90 percent by weight and preferably in the range of about 30 to about 80 percent by weight of the composition.

Operating conditions employed in the process of the present invention are important. Such conditions as temperature, pressure, space velocity, molar ratio of the reactants, and the presence of any diluents will have important effects on the process.

The process of this invention is conducted such that isomerization of the monocyclic alkyl aromatic hydrocarbon is carried out in the vapor-phase by contact in a reaction zone, such as, for example, a fixed bed of catalyst composition, under isomerization effective conditions, said catalyst composition being characterized, as synthesized, as comprising the above-defined zeolite which has been hydrogen or hydrogen-precursor exchanged. This process may be conducted in fixed, moving or fluid bed operation with attendant benefits of either operation readily obtainable.

The present isomerization process may be carried out at a temperature between about 200°C and 450°C and at pressures ranging from about 15 psig up to about 500 psig. The weight hourly space velocities (WHSV) may be from about 0.1 to about 20. Within these limits the conditions of temperature and pressure will vary considerably depending upon equilibrium considerations and type of feed material. Optimum conditions are those in which a maximum yield of the desired isomer products is obtained and hence considerations of temperature and pressure will vary within a range of conversion levels designed to provide the highest selectivity and maximum yield.

4

The starting feed materials for isomerization to be employed in the process of the invention are preferably single ring aromatic hydrocarbons containing 2 to 4 alkyl group substituents on the ring. Typical starting feeds contain 0-100% meta-xylene, 0-~30% ethylbenzene and 0 to 3% ortho + para-xylene. In the process, near-equilibrium mixtures of xylene isomers are obtained with 0-~90% ethylbenzene conversion, depending on the specific reaction conditions. Keeping all other variables constant, ethylbenzene conversion is very sensitive to the change in temperature, being close to 0 at ~ 240°C or below and almost complete above ~350°C. The amount of gas and the apparently accompanying $C_9^+$ products and coke formed, correlate with ethylbenzene conversion, hence are also detected and drastically increased above ~240°C.

The following examples illustrate the production of efficient selective xylene isomerization catalysts and an isomerization reaction. They by no means limit the scope of the invention.

Example 1:

A catalyst given the code CM-11-3 is prepared in the following manner:
Solution A is prepared by dissolving aluminum turnings (1.0 g, 37 mmol) in a solution of sodium hydroxide (1.8 g, 45 mmol) in water (5.0 g, 278 mmol). The dissolution should occur gently, first at room temperature, then at higher temperatures. After complete dissolution, evaporation is carried out to give a final solution of 7.0 g. Solution B is prepared by partial dissolution of silica gel (Davison, grade 950, 60-200 mesh, 43.4 g, 723 mmol) in tetrapropylammonium hydroxide (Fluka, 20% in water, 109.4 g: 107.8 mmol $Pr_4NOH$, 4.86 mol $H_2O$), by heating, thereby evaporating to a final weight of 131.0 g.

Solution A is added dropwise to solution B under efficient stirring and the mixture obtained, which shows an amorphous precipitate in addition to the excess (undissolved) silica, is allowed to stay under stirring for additional period of ~ 0.5 h. It is then introduced into a Teflon-lined autoclave and heated to 155°C for 6 days. The obtained material is filtered off, washed with deionized water and dried at 110°C for ~ 18 h. The white solid is calcined at 540°C (heating rate,~3°/min) for at least about 3 h. Then, it is ion exchanged twice with 1N ammonium chloride solution for 2 h (10 ml solution per 1 g solid), filtered, washed with deionized water until chloride-free, and dried at 110°C for a few hours.

Example 2:

A catalyst given the code CM-11-5 was synthesized as in Example 1, but with solution A prepared by dissolving sodium aluminate (BDH, 3.0 g: $Al_2O_3$ -1.2 g, 11.8 mmol, $Na_2O$ - 1.0 g, 16 mmol, $H_2O$ - 0.81 g, 45 mmol) in a solution of sodium hydroxide (0.32 g, 8 mmol) in water (3.5 g, 194 mmol).

Example 3:

A catalyst given the code CM-11-7 was synthesized as in Example 2, but the material obtained from the autoclave after crystallization was evaporated to give a paste. The latter was dried at 110°C, calcined at 540°C and ion exchanged with ammonium chloride solution as above.

Example 4:

A catalyst given the code CM-112 was synthesized following the conditions and reactant proportions as in Example 3, but with half the amount of tetrapropylammonium hydroxide solution. After calcination, a portion of the obtained material was ion exchanged with ammonium chloride solution as above. Some of the obtained product after calcination was crushed to a fine powded before ion exchange. After drying the $NH_4^+$ form obtained, this zeolite was pelletized with an equal amount of bentonite and calcined at 550°C for 5 h (heating rate 10°/min). It was given the code CM-112A.

Example 5:

A catalyst given the code CM-113 was synthesized as follows: silica gel (Davison, 8.7 g, 145 mmol) was added to tetrapropylammonium hydroxide solution (Fluka, 20% in water, 10.9 g, 10.7 mmol Pr$_4$NOH, 484 mmol H$_2$O) in a 50 ml polypropylene jar. The jar was closed and shaken well. To the obtained mixture solution A was rapidly added, containing sodium aluminate (BDH, 0.6 g, 2.35 mmol Al$_2$O$_3$, 3.2 mmol Na$_2$O, 9 mmol H$_2$O), soldium hydroxide (0.06 g, 1.5 mmol) and water (0.7 g, 38.9 mmol). The jar was then shaken vigorously and placed in the oven at 95°C for 8 days. The content of the jar was mixed with bentonite binder the amount of which was regulated to afford a wet paste that was then shaped, dried, calcined and ion exchanged as above.

Example 6:

A catalyst given the code CM-131 was synthesized as follows:

An aluminate solution was prepared by dissovling 13.54 g sodium aluminate (BDH, 53 mmol Al$_2$O$_3$, 72 mmol Na$_2$O, 20.3 mmol H$_2$O) in a solution of 1.44 g sodium hydroxide (36 mmol) in 15.8 g water (878 mmol).

A silicate mixture was prepared by adding 394.5 g of silica gel (Davison, grade 950, 6.575 mol) to 500 ml tetrapropylammonium hydroxide (Fluka, 20% in water, 492.6 mmol Pr$_4$NOH, 22.222 mol H$_2$O) in a 1 liter polypropylene jar.

The aluminate solution was poured slowly into the silicate mixture, under gentle agitation, and a homogenous mixture was obtained. After equilibrating the mixture at room temperature for ~ 1 h, the jar was introduced into the oven and allowed to stay at 95°C for 7 days. At the middle of this period the content of the jar was gently agitated to remove gas bubbles. CM-131B is a portion of the obtained material after drying, calcining and ion exchanging as above.

The following Tables describe by way of typical examples not meant to limit the scope of the present invention, the effectivity of catalysts obtained in the above Examples in the conversion of xylene mixtures rich in the meta isomer to mixtures containing relatively large quantities of the other isomers, i.e. ortho and para.

Experiments were done in a 20 mm (I.D.) Pyrex tubular reactor surrounded by electrical heater equiped with a thermoregulator. The catalyst (0.5 - 8 g) was preactivated at 500°C for 2 h under a flow of air, 60 ml/min then at 450°C for 0.5 h under Ar at the same flow-rate. Heating was then adjusted to reach the required reaction temperature and the flow rate of Ar, to be used later as carrier gas, was reduced to 30 ml/h. At this point a Sage syringe pump was operated to feed the reactant into the reactor at a predetermined flow rate. Samples were taken from the bottom exit of a cold-water condenser placed below the reactor, and analyzed by gas chromatography over typical columns for aromatics separation.

Table 1 shows typical results with CM-11-3 using different feeds. Isomerization proceeds quantitatively at relatively high temperatures and high conversions of ethylbenzene with practically no xylene degradation. Tables 2 and 3 show that CM-11-5 is also an efficient selective xylene isomerization catalyst. Table 3 presents a wider product analysis. The process was carried out at ~300°C. In Table 4 catalysts CM-113 and CM-112A are shown to be effective at temperatures in the range 230-270°C.

Unlike the former experiments, the experiments described in Table 4 were performed without carrier gas. Table 5 present results obtained with CM-131B, showing that this catalyst is also highly effective and selective for the isomerization of xylene at moderate temperatures.

Table I[a]

| Exp. | B52 | | H66 | | H67 | | H68 | | H69 | | H70 | |
|---|---|---|---|---|---|---|---|---|---|---|---|---|
| Feed[b] | MX | | A | | A | | A | | A | | A | |
| WHSV, h$^{-1}$ | 1.26 | | 0.6–0.5 | | 0.67 | | 1.44 | | 1.44 | | 0.225 | |
| T, °C | 350 | | 440 | | 400 | | 440 | | 480 | | 440 | |
| Onstream time, h | 0.5 | 2.0 | 0.5 | 2.0 | 1.0 | 2.5 | 0.5 | 3.0 | 0.5 | 3.0 | 0.5 | 2.5 |
| Analysis[c] | | | | | | | | | | | | |
| B | 0.1 | – | 7.9 | 8.3 | 6.5 | 10.3 | 8.2 | 7.4 | 9.1 | 6.9 | 6.7 | 5.3 |
| T | 1.6 | 1.2 | 4.4 | 4.6 | 2.1 | 2.6 | 3.5 | 3.8 | 6.3 | 5.6 | 4.8 | 3.7 |
| EB | | | 2.2 | 2.7 | 7.7 | 5.9 | 6.4 | 6.1 | 3.7 | 3.9 | 4.1 | 2.8 |
| PX | 20.9 | 21.6 | 10.6 | 13.2 | 16.0 | 13.3 | 15.7 | 15.5 | 14.5 | 14.8 | 16.0 | 15.8 |
| MX | 59.1 | 58.3 | 41.2 | 42.4 | 44.1 | 36.7 | 45.8 | 42.1 | 42.2 | 44.3 | 45.6 | 46.2 |
| OX | 16.7 | 17.5 | 29.0 | 24.8 | 16.9 | 18.2 | 16.3 | 18.8 | 19.0 | 20.7 | 16.8 | 21.6 |
| ΣX | 96.7 | 97.4 | 80.8 | 80.4 | 77.0 | 68.0 | 77.8 | 76.4 | 75.7 | 79.8 | 78.5 | 83.6 |
| ET | 1.5[d] | 1.4[d] | 3.0 | 2.2 | 2.3 | 2.0 | 1.9 | 2.1 | 3.0 | 2.6 | 3.4 | 2.7 |
| TMB | | | 1.8 | 1.9 | 2.7 | 3.3 | 1.5 | 3.0 | 1.1 | 0.7 | 1.7 | 1.6 |
| Isomer composition,% | | | | | | | | | | | | |
| Para | 21.6 | 22.1 | 13.1 | 16.4 | 20.7 | 19.4 | 20.1 | 20.2 | 19.2 | 18.5 | 20.3 | 18.9 |
| Meta | 61.1 | 59.9 | (51.0) | (52.7) | 57.3 | 53.8 | 58.9 | 55.1 | 55.7 | 55.5 | 58.1 | 55.2 |
| Ortho | 17.3 | 18.0 | (35.9) | (30.8) | 21.9 | 26.7 | 20.9 | 24.6 | 25 | 25.4 | 21.5 | 25.8 |

[a] 1 g. [b]A: EB, 22.6%; PX, 4.0%; MX, 70.2%; OX, 3.3%.

[c] B= benzene, T= toluene, EB= ethylbenzene, PX= para-xylene, MX= meta-xylene, OX= ortho-xylene, ΣX= sum of xylenes ET= ethyltoluene, TMB= trimethylbenzene.

[d] C$_9^+$

Table 2[a]

| Time on stream, h | 1 | 2 | 3 |
|---|---|---|---|
| Analysis[b] | | | |
| B | 1.15 | 0.82 | 0.72 |
| T | 0.36 | 0.26 | 0.76 |
| EB | 29.3 | 26.7 | 27.1 |
| PX | 10.5 | 13.3 | 14.6 |
| MX | 48.2 | 45.9 | 43.6 |
| OX | 8.1 | 10.4 | 11.1 |
| $C_9^+$ | 1.15 | 1.65 | 1.63 |
| EB conversion, % | 3.0 | 11.6 | 10.3 |
| X degradation | 3.6 | 0 | 0.2 |
| | | | |
| Isomer composition, % | | | |
| Para | 15.7 | 19.2 | 21.1 |
| Meta | 72.1 | 66.0 | 62.9 |
| Ortho | 12.2 | 14.9 | 16.1 |

[a] Exp. H115. Feed, T, 0.02%; EB, 30.22%; PX, 1.69%; MX, 66.98%; OX, 0.74%, 1 g cat. CM-11-5, 300°C, flow -0.5 ml/h.

[b] for abbreviations, see Table 1

## Table 3[a]

Time on stream, h

| Product Analysis[b], wt% | 1 | 2 | 3 | 4 | 5 |
|---|---|---|---|---|---|
| B | 1.61 | 1.60 | 1.44 | 1.43 | 1.43 |
| T | 0.61 | 0.57 | 0.48 | 0.43 | 0.43 |
| EB | 25.1 | 25.8 | 25.8 | 25.7 | 26.1 |
| Xylene | | | | | |
| Para | 13.4 | 14.4 | 14.8 | 14.9 | 14.5 |
| Meta | 45.9 | 43.9 | 41.5 | 43.5 | 43.3 |
| Ortho | 10.1 | 10.7 | 13.0 | 11.0 | 11.3 |
| Ethyltoluene | | | | | |
| Para | 0.16 | 0.14 | 0.13 | 0.13 | 0.20 |
| Meta | 0.15 | 0.11 | 0.10 | 0.11 | 0.10 |
| Ortho | 0.03 | -- | -- | -- | -- |
| $C_9^+$ | | | | | |
| Mesitylene | 0.51 | 0.56 | 0.54 | 0.53 | 0.12 |
| Pseudocumene | 1.25 | 1.21 | 1.27 | 1.29 | 0.55 |
| Durene | 0.75 | 0.74 | 0.69 | 0.66 | 1.72 |
| EB conversion, % | 16.9 | 14.6 | 14.6 | 15.0 | 13.6 |
| X degradation, % | 0.1 | 0.6 | 0.2 | 0 | 0.4 |
| X isomer composition, % | | | | | |
| Para | 19.3 | 20.9 | 21.3 | 21.5 | 20.9 |
| Meta | 66.2 | 63.6 | 59.9 | 62.6 | 62.7 |
| Ortho | 14.5 | 15.5 | 18.8 | 15.9 | 16.4 |

[a] Exp. H116. Feed, as in Table 2, 1 g cat. CM-11-5, 325°C flow-0.5 ml/h.

[b] for abbreviations, see Table 1.

Table 4[a]

| Catalyst | CM-113 | | CM-112A | | |
|---|---|---|---|---|---|
| Cat. weight, g | 5.5 | | 8 | | |
| Feed[a] | B | | C | | |
| Temp., °C | 260 | 270 | 230 | 255 | 270 |
| Analysis[b] | | | | | |
| B | 0.7 | 1.0 | -- | 0.6 | 1.3 |
| T | 0.1 | 0.1 | -- | 0.1 | 0.2 |
| EB | 20.3 | 18.6 | 28.2 | 25.9 | 25.6 |
| PX | 15.4 | 16.2 | 7.4 | 14.5 | 14.3 |
| MX | 52.2 | 44.8 | 61.7 | 44.7 | 46.5 |
| OX | 8.9 | 12.5 | 2.7 | 8.9 | 8.4 |
| $C_9^+$ | 2.7 | 5.8 | -- | 3.2 | 2.3 |
| EB Conversion | 8.8 | 16.4 | -- | 8.1 | 9.2 |
| X Degradation | 1.5 | 5.4 | -- | 4.0 | 3.6 |
| Isomer composition % | | | | | |
| Para | 20.1 | 22.0 | 10.3 | 21.3 | 20.7 |
| Meta | 68.2 | 61.0 | 85.9 | 65.6 | 67.2 |
| Ortho | 11.6 | 17.0 | 3.8 | 13.1 | 12.1 |

[a] B: EB, 22.27%; PX, 1.68%; MX, 74.9%; OX, 1.2%.

C: EB, 28.6%; PX, 7.4%; MX, 61.7%; OX, 2.7%

[b] after 1 h on stream; for abbreviations, see Table 1.

Table 5[a]

| Temp, $^{\circ}$C | 300 | 340 | 380 |
|---|---|---|---|
| Analysis[b] | | | |
| B | 2.8 | 4.22 | 7.36 |
| T | 0.9 | 0.93 | 2.17 |
| EB | 18.9 | 15.8 | 11.6 |
| PX | 15.8 | 16.7 | 16.2 |
| MX | 45.7 | 41.8 | 40.3 |
| OX | 12.8 | 15.1 | 15.1 |
| $C_9^+$ | 2.8 | 5.0 | 6.8 |
| EB conversion, % | 21.7 | 34.5 | 52.0 |
| X degradation, % | 0.9 | 1.9 | 5.2 |
| Isomer composition, % | | | |
| Para | 21.3 | 22.7 | 22.6 |
| Meta | 61.4 | 56.8 | 56.3 |
| Ortho | 17.3 | 20.5 | 21.1 |

[a] Feed: EB, 22.27%; PX, 1.68%; MX, 74.9%; OX, 1.2%, 4 g catalyst CM-131B; time on stream, 1 h; flow, 2 ml/h, no carrier gas.

[b] for abbreviation see Table 1.

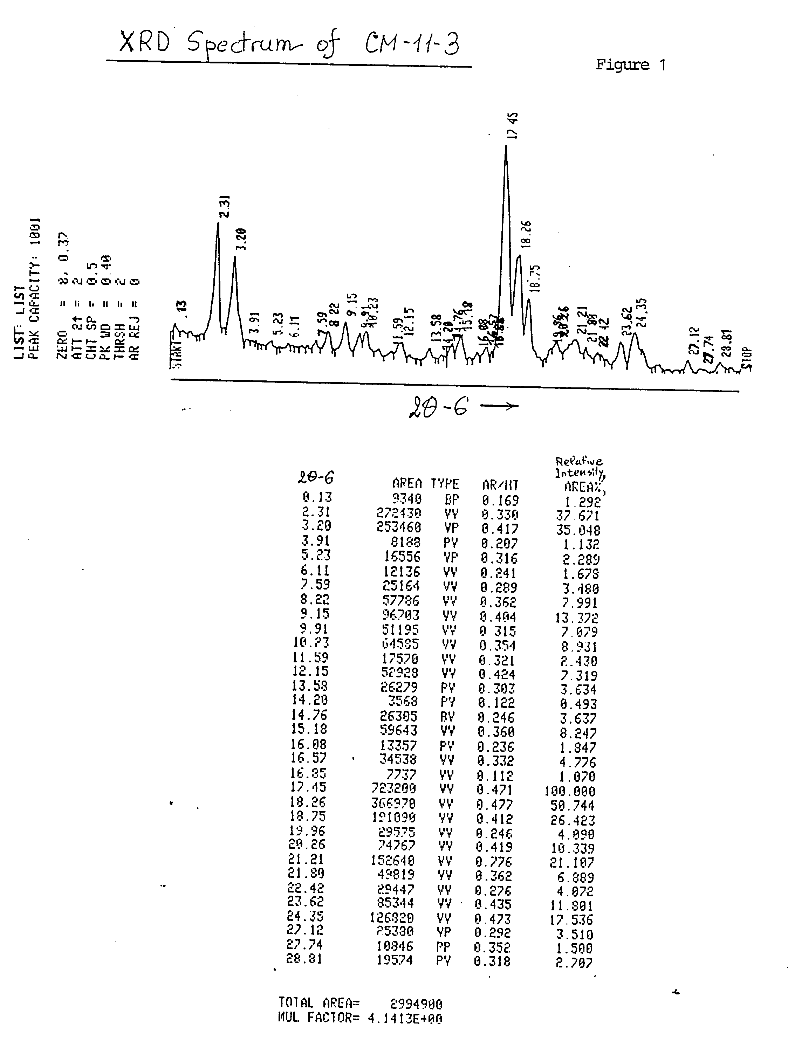

## Claims

1. A crystalline aluminosilicate zeolite catalyst having a silica/alumina mole ratio of greater than 12, being the product of the reaction of a gel consisting of $Na_2O$/(propyl)$_4$NOH/$Al_2O_3$/$SiO_2$/$H_2O$, wherein the $SiO_2$-(propyl)$_4$NOH ratio is 5 to 20, preferably about 10, and the $H_2O$/$SiO_2$ ratio is in the range of 1 to 10, preferably about 5; said catalyst being prepared by the dissolution of sodium hydroxide in water, addition of aluminum or sodium aluminate and dissolution thereof; preparing a silica solution by dissolving $SiO_2$ in a tetrapropylammonium hydroxide (20%) solution; adding the aluminate solution to the thus obtained silicate solution, heating same in an autoclave between 90°C and 160°C, filtering the product, washing same, drying same at about 100°C, heating same at about 500-580°C for a number of hours, and effecting an ion exchange with a 1N ammonium chloride solution and drying above 100°C.

2. A catalyst according to claim 1, wherein the material obtained from the oven is evaporated to form a viscous material, to which a binder is added, which is dried as obtained or after shaping, at a temperature of from 90°C to 150°C, before calcination.

3. A catalyst according to claim 1 or 2, wherein the calcination is effected to about 540°C for at least 1 hour and where the ion-exchanged material is activated by heating at a temperature of 500°C.

4. A process for the selective isomerization of xylenes which comprises contacting the xylene starting material wherein the starting material comprises 60 to 100 per cent by weight of meta-xylene, the balance being ethylbenzene, p-xylene and o-xylene in the vapor phase at a temperature between 200°C and about 450°C with a catalyst defined in claim 1, at a weight hourly space velocity of from about 0.1 to about 1.0 where the product comprises mainly an isomeric mixture of xylene close to its equilibrium composition.

5. A process according to claim 4, whenever effected at atmospheric pressure.

6. A process as claimed in claims 4 or 5, wherein the isomerization is effected between 200°C and 300°C.

7. A process according to any of claims 4 to 6, wherein the isomerization is effected in a fixed bed reactor.

# XRD Spectrum of CM-11-3

Figure 1

LIST: LIST
PEAK CAPACITY: 1001

ZERO = 8, 0.37
ATT 2↑ = 2
CHT SP = 0.5
PK WD = 0.40
THRSH = 2
AR REJ = 0

2θ-6 →

| 2θ-6 | AREA | TYPE | AR/HT | Relative Intensity, AREA% |
|---|---|---|---|---|
| 0.13 | 9340 | BP | 0.169 | 1.292 |
| 2.31 | 272430 | VV | 0.330 | 37.671 |
| 3.20 | 253460 | VP | 0.417 | 35.048 |
| 3.91 | 8188 | PV | 0.207 | 1.132 |
| 5.23 | 16556 | VP | 0.316 | 2.289 |
| 6.11 | 12136 | VV | 0.241 | 1.678 |
| 7.59 | 25164 | VV | 0.289 | 3.480 |
| 8.22 | 57786 | VV | 0.362 | 7.991 |
| 9.15 | 96703 | VV | 0.404 | 13.372 |
| 9.91 | 51195 | VV | 0.315 | 7.079 |
| 10.23 | 64585 | VV | 0.354 | 8.931 |
| 11.59 | 17570 | VV | 0.321 | 2.430 |
| 12.15 | 52928 | VV | 0.424 | 7.319 |
| 13.58 | 26279 | PV | 0.303 | 3.634 |
| 14.20 | 3568 | PV | 0.122 | 0.493 |
| 14.76 | 26305 | RV | 0.246 | 3.637 |
| 15.18 | 59643 | VV | 0.360 | 8.247 |
| 16.08 | 13357 | PV | 0.236 | 1.847 |
| 16.57 | 34538 | VV | 0.332 | 4.776 |
| 16.85 | 7737 | VV | 0.112 | 1.070 |
| 17.45 | 723200 | VV | 0.471 | 100.000 |
| 18.26 | 366970 | VV | 0.477 | 50.744 |
| 18.75 | 191090 | VV | 0.412 | 26.423 |
| 19.96 | 29575 | VV | 0.246 | 4.090 |
| 20.26 | 74767 | VV | 0.419 | 10.339 |
| 21.21 | 152640 | VV | 0.776 | 21.107 |
| 21.89 | 49819 | VV | 0.362 | 6.889 |
| 22.42 | 29447 | VV | 0.276 | 4.072 |
| 23.62 | 85344 | VV | 0.435 | 11.801 |
| 24.35 | 126820 | VV | 0.473 | 17.536 |
| 27.12 | 25380 | VP | 0.292 | 3.510 |
| 27.74 | 10846 | PP | 0.352 | 1.500 |
| 28.81 | 19574 | PV | 0.318 | 2.707 |

TOTAL AREA= 2994900
MUL FACTOR= 4.1413E+00

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 186 480 (EXXON) --- | | B 01 J 29/28 C 07 C 5/27 |
| A | FR-A-2 504 510 (NATIONAL DISTILLERS) * Pages 6,7 * --- | | |
| A,D | US-A-3 702 886 (R.J. ARGAUER) * Example 6; column 9 * --- | | |
| A | DE-A-2 817 577 (MOBIL OIL) --- | | |
| A | GB-A-1 161 974 (MOBIL OIL) ----- | | |

TECHNICAL FIELDS SEARCHED (Int. Cl.4)

B 01 J 29/00

The present search report has been drawn up for all claims

| Place of search | Date of completion of the search | Examiner |
|---|---|---|
| THE HAGUE | 02-12-1987 | DEVISME F.R. |

EPO FORM 1503 03.82 (P0401)